# EUROPEAN PATENT SPECIFICATION

(11) **EP 2 449 951 B1**
(45) Date of publication and mention of the grant of the patent: **21.12.2016**
(21) Application number: 11186910.3
(22) Date of filing: 27.10.2011
(51) Int. Cl.: A61B 1/00, A61B 1/06

(54) **Endoscopic apparatus**
Endoskopische Vorrichtung
Appareil endoscopique

(30) Priority: 09.11.2010 JP 2010251301
(43) Date of publication of application: 09.05.2012
(73) Proprietor: Fujifilm Corporation, Minato-ku Tokyo (JP)
(72) Inventor: Seto, Yasuhiro, Kanagawa (JP)
(74) Representative: Klunker . Schmitt-Nilson . Hirsch

(56) References cited:
- EP-A1- 2 030 559
- US-A1- 2005 099 824

## Description

### BACKGROUND OF THE INVENTION

### 1. Technical Field

The present invention relates to an endoscopic apparatus

### 2. Description of Related Art

Endoscopic apparatus for observing tissues in a body cavity has been known broadly. Generally, an endoscopic apparatus has a configuration in which white light emitted from a white light source such as a xenon lamp is supplied as illumination light to a to-be-observed region in a body cavity through a light guide and an image based on reflected light from the to-be-observed region irradiated with the white light is captured by an imaging device to generate an observation image. In recent years, an endoscopic apparatus having observation modes using special light is also being used. The observation modes are narrow-band light observation for observing blood capillaries or microscopic structures in a tissue surface layer while biological tissues are irradiated with narrow-band light with a specific wavelength, fluorescent observation using intrinsic fluorescence or drug fluorescence, etc.

A light source for irradiation with special light in the endoscopic apparatus may have a configuration in which light from a white light source such as a xenon lamp is made to pass through a filter so that light with a desired wavelength band is extracted, or a configuration in which wavelength of light emitted from a laser light source or a semiconductor light emitting device such as a light emitting diode is used as it is (for example, see JP-A-2005-319115).

EP 2 030 559 A1 discloses an endoscopic apparatus in which the light source control unit is adapted to vary the integral light volume of the illumination light by one of three modulation control modes, i. e. a pulse number modulation (PNM) in which the pulse width of the drive current and the amplitude thereof are constant, a pulse width modulation (PWM), in which the pulse number and the amplitude of the pulses is constant within an exposure time period, and a pulse amplitude modulation (PAM), in which the number of pulses and the pulse width are constant within a predetermined exposure time period. Any of these three modulation schemes may be used.

### SUMMARY

When a white light source such as a xenon lamp is used as a light source of an endoscopic apparatus, a slit is disposed on the way of an optical path of outgoing light so that the quantity of light is controlled by light shielding with the slit. According to this configuration, a dynamic range of about 2400:1 for control of the light quantity can be achieved. On the other hand, when a semiconductor light source is used for controlling light quantity, generally, the light quantity is often controlled by increase/decrease of a driving current or modulation of pulse width. By control of the light quantity only in this manner, there is a limit for securing a wide dynamic range or achieving a high light quantity resolution. It is therefore difficult to control the quantity of light emission desirably with high accuracy. Although a narrow pulse generator or a high-resolution type PWM controller may be used to control the light quantity with high accuracy, each of the devices is too expensive to be mounted on an endoscopic apparatus practically. In addition, the semiconductor light source has a characteristic in that the light quantity may fluctuate in some use condition due to temperature dependency in intensity of light emission. In fact, therefore, there are still many problems as to how control the light quantity of the semiconductor light source as equivalently as or more equivalently than the white light source such as a xenon lamp.

An object of the invention is to provide an endoscopic apparatus in which a wide dynamic range and high light quantity resolution can be secured even with illumination light from a semiconductor light source so that the light quantity can be controlled with high accuracy.

The invention has the configuration as defined by claim 1.

An endoscopic apparatus emits illumination light with a desired light quality from a front end of an endoscope insertion portion. The endoscopic apparatus includes a semiconductor light source, an imaging unit and a light source control unit. The semiconductor light source generates the illumination light. The imaging unit adjusts an exposure time by using an electronic shutter. The light source control unit generates driving pulses to drive and light the semiconductor light source in accordance with an inputted target light quality. The light source control unit performs first pulse modulation control, second pulse modulation control and third pulse modulation control in descending order of the target light quantity. In the first pulse modulation control, the number of the driving pulses is reduced to shorten a lighting time of the semiconductor light source to a predetermined lighting time for an exposure time of an electronic shutter within one frame. In the second pulse modulation control, the driving pulses are thinned at predetermined intervals in a predetermined lighting time in a region where the first pulse modulation control is performed so that pulse density is reduced in the lighting time. In the third pulse modulation control, pulse width of each of the driving pulses whose number is minimized in a region where the second pulse modulation control is performed is reduced.

According to an endoscopic apparatus of the invention, a wide dynamic range and high light quantity resolution can be secured even when illumination light from a semiconductor light source is used. Thus, the intensity of the illumination light in normal observation or special light observation can be set desirably.

### BRIEF DESCRIPTION OF THE DRAWINGS

Fig. 1 is a view for explaining an embodiment of the invention, showing a configuration of an endoscopic apparatus having an endoscope and units the endoscope is connected to.
Fig. 2 is an external view showing a specific example of the configuration of the endoscopic apparatus.
Fig. 3 is a block diagram of control performed by an imaging signal processing portion.
Fig. 4 is a timing chart of an example for controlling driving pulses.
Fig. 5 is a graph showing contents of pulse control on light quantity ranging from maximum light quantity to minimum light quantity.
Fig. 6 is a block configuration diagram schematically showing a main portion configuration of a light source unit and optical paths for guiding light to light irradiation windows.
Fig. 7 is a graph showing a spectrum of illumination light generated from light emitted from the light source unit.
Fig. 8 is an explanatory view showing a light quantity control pattern in combination of a single-lamp system and a dual-lamp system.

### DETAILED DESCRIPTION OF EMBODIMENTS OF THE INVENTION

An embodiment of the invention will be described below in detail with reference to the drawings.

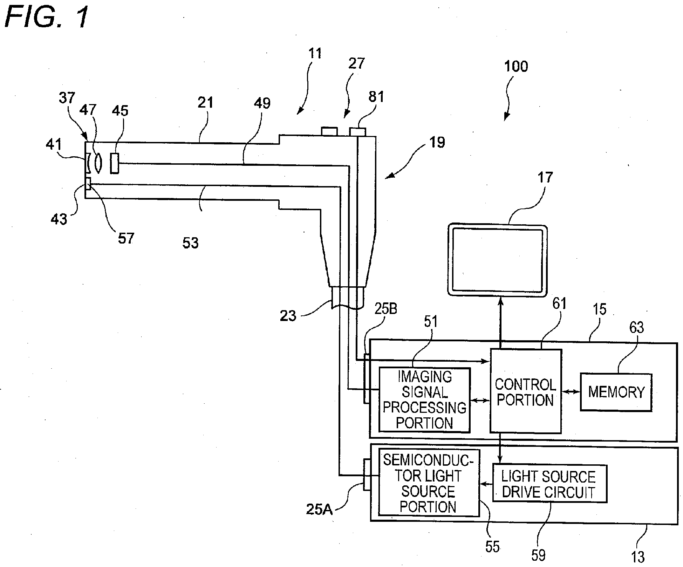

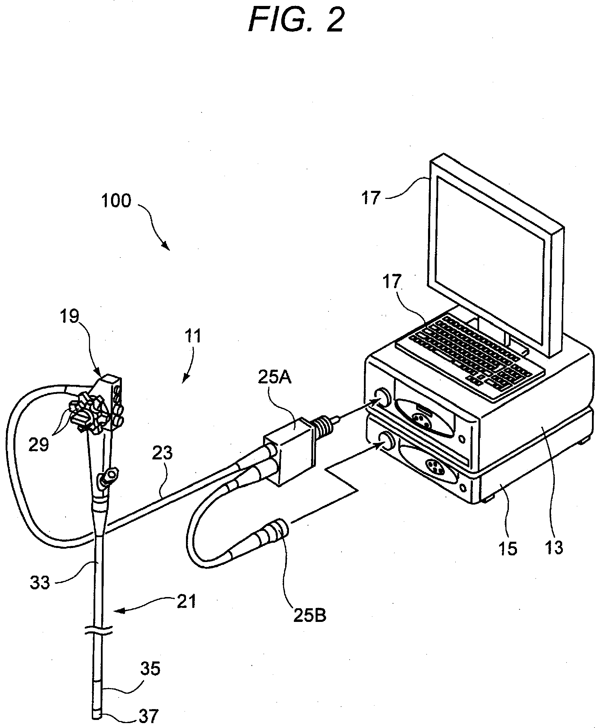

Fig. 1 is a view for explaining an embodiment of the invention, showing a configuration of an endoscopic apparatus having an endoscope and units the endoscope is connected to. Fig. 2 is an external view showing a specific example of the configuration of the endoscopic apparatus.

As shown in Fig. 1, an endoscopic apparatus 100 has an endoscope 11, a light source unit 13, a processor 15 for performing signal processing on a captured image, and a monitor 17. The endoscope 11 has a body operation portion 19 and an insertion portion 21 which is connected to the body operation portion 19 in order to be inserted into a subject (body cavity). A universal cable 23 is connected to the body operation portion 19. A front end of the universal cable 23 is connected to the light source unit 13 through a light guide (LG) connector 25A. In addition, an imaging signal is inputted to the processor 15 through a video connector 25B.

As shown in Fig. 2, a pair of angle knobs 29 as well as various operation buttons 27 such as buttons for sucking, feeding air and feeding water in the front end side of the insertion portion 21, a shutter button for capturing an image, etc. are provided in the body operation portion 19 of the endoscope 11.

The insertion portion 21 is constituted by a soft portion 33, a bendable portion 35 and a front end portion (endoscope front end portion) 37 in ascending order of distance from the body operation portion 19. When the angle knobs 29 of the body operation portion 19 are turned, the bendable portion 35 is remotely operated to be bent. Thus, the front end portion 37 can be steered in a desired direction.

In addition, as shown in Fig. 1, an observation window 41 of an imaging optical system and a light irradiation window 43 of an illumination optical system are disposed in the front end portion 37 of the endoscope 11 so that light reflected by a subject irradiated with illumination light from the light irradiation window 43 can be captured through the observation window 41. An observation image captured thus is displayed on the monitor 17 connected to the processor 15.

In this embodiment, the imaging optical system includes an imaging device 45 such as a CCD (Charge Coupled Device) or a CMOS (Complementary Metal-Oxide Semiconductor) and an optical member such as an imaging lens 47. An observation image taken in by the imaging optical system is focused on a light reception surface of the imaging device 45 and converted into an electric signal. The electric signal is inputted to an imaging signal processing portion 51 of the processor 15 through a signal cable 49, and converted into a video signal in the imaging signal processing portion 51.

On the other hand, the illumination optical system includes the light source unit 13, an optical fiber 53, and a wavelength conversion portion 57. The light source unit 13 has a semiconductor light source 55. The optical fiber 53 is connected to the light source unit 13. The wavelength conversion portion 57 is disposed on the light outgoing side of the optical fiber 53. The optical fiber 53 guides laser light to the endoscope front end portion 37 so as to generate white illumination light in which light emitted from the wavelength conversion portion 57 of the front end portion 37 is combined with the laser light. The wavelength conversion portion 57 is provided with fluorescent substances which are excited by the laser light to thereby emit light. The semiconductor light source 55 emits light with desired intensity in response to a driving signal from a light source drive circuit 59 based on an instruction from a control portion 61.

The control portion 61 is connected to a memory 63 as a storage unit for storing imaging signals and various information, so that image data outputted from the imaging signal processing portion 51 can be displayed on the monitor 17. The control portion 61 is connected to a not-shown network such as a LAN to distribute information including image data. Thus, the control portion 61 controls the endoscopic apparatus 100 as a whole.

The semiconductor light source 55 has a semiconductor laser which emits blue light with a center wavelength of 445 nm (the semiconductor light source will be also hereinafter referred to as laser light source). The semiconductor light source 55 emits blue laser light while the quantity of the emitted light is controlled. The emitted light is irradiated to the wavelength conversion portion 57 of the insertion portion 21 of the endoscope through the optical fiber 53. For example, a broad-area type InGaN-based laser diode may be used as the semiconductor light source 55.

The wavelength conversion portion 57 includes a plurality of kinds of fluorescent substances (such as YAG-based fluorescent substances or fluorescent substances containing BAM (BaMgAl₁₀O₃₇) etc.) which can absorb a part of the laser light from the semiconductor light source 55 so as to be excited to emit green to yellow light. Thus, the laser light from the semiconductor light source 55 is combined with the excited green to yellow light obtained by conversion from the laser light, so as to generate white light.

Next, description will be made on the procedure in which the endoscopic apparatus 100 configured thus makes control to increase/decrease the intensity of light emitted from the semiconductor light source 55.

The imaging signal processing portion 51 provided in the processor 15 shown in Fig. 1 receives RAW data outputted by the imaging device 45 of the endoscope 11 connected to the processor 15, and outputs a control signal for controlling the semiconductor light source 55 to the light source drive circuit 59 to thereby optimize the quantity of illumination light in accordance with the RAW data.

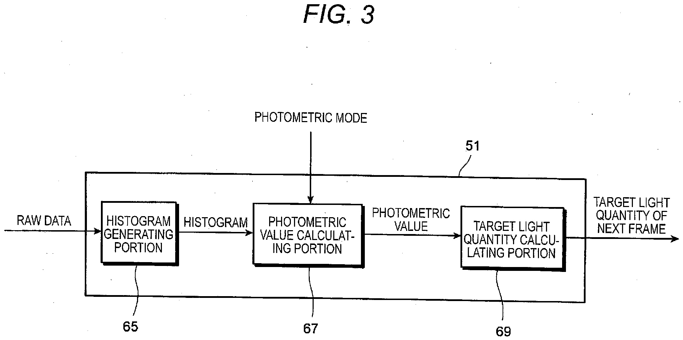

Fig. 3 shows a block diagram of control performed by the imaging signal processing portion 51. The RAW data (information of a raw image) outputted from the imaging device 45 is inputted to the imaging signal processing portion 51. A histogram generating portion 65 generates a histogram of light quantity corresponding to the RAW data and supplies the generated histogram to a photometric value calculating portion 67. The photometric value calculating portion 67 calculates a photometric value based on the supplied histogram and brightness detection values obtained by various photometric modes (peak value, mean value, etc.). A target light quantity calculating portion 69 obtains a target light quantity of the next frame in accordance with the calculated photometric value. Here, the target light quantity has a value corresponding to an F-number of the background-art white light source such as a xenon lamp. The target light quantity is, for example, expressed in 12-bit gradation (0 to 4096).

Next, the target light quantity is converted into a signal indicating the quantity of light emitted by the semiconductor light source 55. The control portion 61 shown in Fig. 1 controls the quantity of light emitted by the semiconductor light source by pulse-lighting control with driving pulses. The driving pulses are generated by the light source drive circuit 59 and inputted to the semiconductor light source 55. Three kinds of controls including PNM (Pulse Number Modulation), PDM (Pulse Density Modulation) and PWM (Pulse Width Modulation) or four kinds of controls including current value control in addition to the aforementioned three kinds of controls are used for controlling the driving pulses. The following description will be made in an example in which the aforementioned four kinds of control are used.

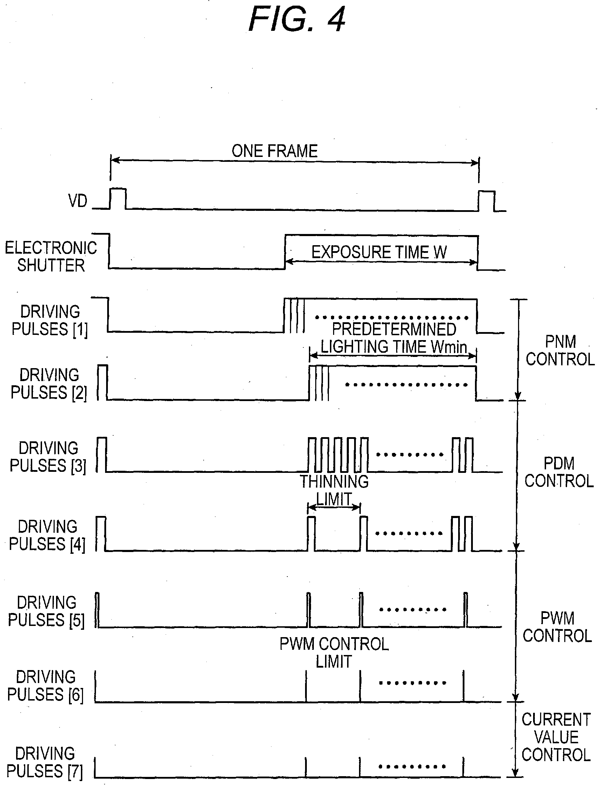

Fig. 4 shows a timing chart of an example for controlling driving pulses. The maximum light quantity is obtained by driving pulses [1] which enable lighting all over an exposure time W of an electronic shutter in a period of one frame of an image defined by a vertical sync signal VD. Here, assume that the period of one frame is 33 ms, and the shutter speed is 1/60 s. In addition, assume that the frequency of the driving pulses [1] is 120 kHz, and 2,000 pulses are included in the exposure time of the electronic shutter.

Assume that the light quantity is decreased from the maximum light quantity provided by the driving pulses [1]. In this case, the PNM control, the PDM control, the PWM control and the current value control are performed in first to fourth pulse modulation regions respectively in descending order of light quantity, so that the light quantity is decreased gradually.

First, in the PNM control, the number of pulses is reduced in a rear-aligned manner in the temporal axis all over the exposure time W of the electronic shutter so that the lighting time is shortened. That is, the number of driving pulses is reduced to delay the drive start timing till the number of driving pulses reaches a predetermined minimum ratio to the exposure time of the electronic shutter in one frame, as shown in driving pulses [2]. Thus, the lighting time of the semiconductor light source 55 is shortened. Incidentally, the maximum light quantity does not have to correspond to lighting all over the exposure time W of the electronic shutter but may correspond to lighting all over the time for one frame or may correspond to continuous lighting.

Next, as shown in driving pulses [3], the lighting time of the semiconductor light source 55 is shortened to a predetermined lighting time Wmin by the PNM control, and the driving pulses are then thinned by the PDM control. In the PDM control, the driving pulses are thinned at predetermined intervals in the lighting time shortened to the predetermined lighting time Wmin, so that the pulse density within the lighting time is reduced.

As shown in driving pulses [4], the PDM control is performed till the pulse interval of the driving pulses reaches a thinning limit, that is, till the driving pulses have a predetermined minimum pulse density.

Next, as shown in driving pulses [5], the pulse width of each driving pulse is reduced by the PWM control after the driving pulses reach a predetermined minimum number of pulses. Then, as shown in driving pulses [6], the PWM control is performed till the pulse width of each driving pulse reaches a PWM control limit, that is, till the pulse width reaches a predetermined minimum pulse width.

Next, as shown in driving pulses [7], the driving current is lowered after each driving pulse reaches the predetermined minimum pulse width. That is, the pulse amplitude of each driving pulse whose pulse width has been made minimal is reduced uniformly.

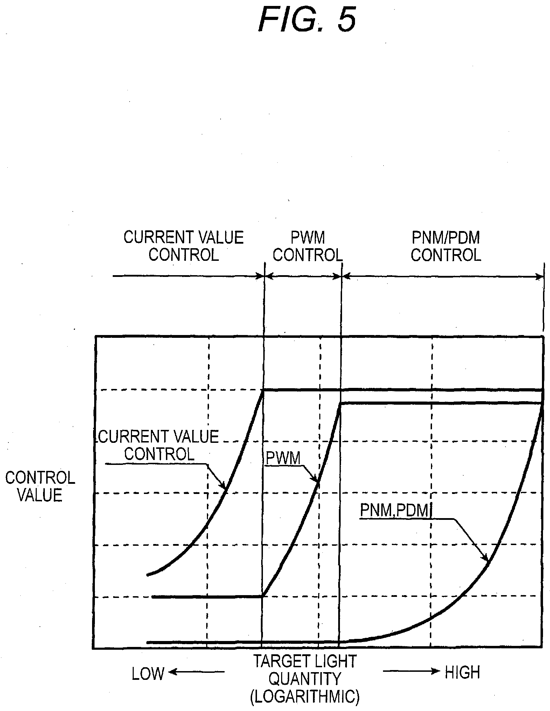

The aforementioned contents of information of control parameters for light quantity ranging from the maximum light quantity to the minimum light quantity are shown in a lump in Fig. 5 and Table 1. The information of control parameters shown in Fig. 5 and Table 1 is stored in the memory 63 shown in Fig. 1, and referred to at any time by the control portion 61 so that desired driving pulses are generated.

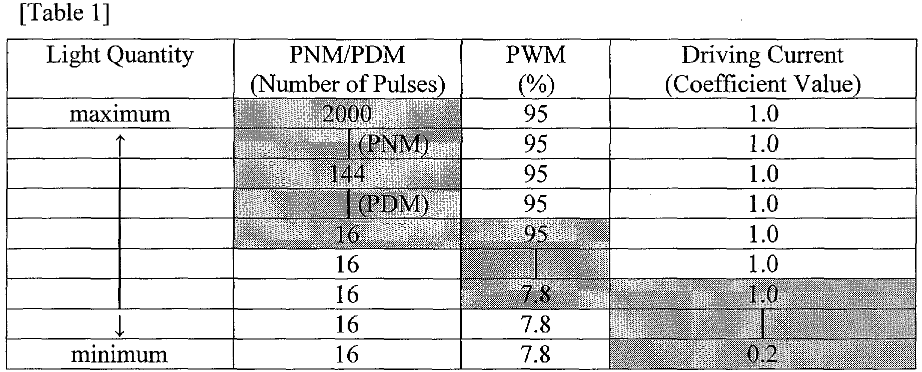

Thus, in performing control to reduce the light quantity, the PNM control is first performed on the maximum light quantity so that it is possible to shorten the lighting time of the semiconductor light source 55 to thereby suppress occurrence of blurring of a captured image caused by shaking. In addition, the non-lighting time of the semiconductor light source 55 is expanded to obtain an effect to reduce heat generated by the light source itself or each optical member on the optical path as compared with the case of continuous lighting.

In addition, control is switched from the PNM control to the PDM control as soon as the lighting time is shortened to the predetermined lighting time. It is therefore possible to keep a moderate lighting time (144 pulses in the aforementioned example) to thereby suppress flicker during observation of a motion picture.

The number of pulses (16 pulses in the aforementioned example) which is a lower limit in the PDM control can prevent the dimming resolution from being roughed by the PDM control.

The PDM control is performed till the thinning limit of the driving pulses, and control is switched from the PDM control to the PWM control to further reduce the target light quantity. In the PWM control, the duty ratio of each driving pulse is changed so that the light quantity can be adjusted more finely in a range of the light quantity which is lower than the thinning limit. Thus, the dimming resolution is improved.

In the pulse lighting control over the semiconductor light source 55, speckle noise may cause unevenness in illumination with the semiconductor light source 55. The speckle noise can be reduced by high frequency modulation driving. In this example of control, driving is always performed with pulses of 120 kHz. An upper limit of the duty ratio in the PWM control is set at 95% in order to obtain a satisfactory effect to reduce the speckle noise.

The lower limit value of the duty ratio is set as follows. That is, real laser light cannot faithfully follow a rising signal for driving, but rises with a certain delay component. In the same manner, the laser light also has a delay component at trailing time. It is therefore expected that each driving pulse will trail before reaching a target value if the driving pulse is extremely narrow in width. Accordingly, the lower limit value of the duty ratio is set at 7.8% in order to perform the PWM control correctly.

Then, the current value control is performed on the driving pulses in a minimum pulse range which is on the minimum light quantity side of the light quantity control, so that the light quantity can be controlled with high accuracy. That is, the outgoing light quantity is reduced with a set light quantity control value multiplied by a coefficient value of 1.0 to 0.2. The current value with which the laser light source begins to emit light is slightly shifted in accordance with the state of heat generated by the light emitting device itself, so as to affect the accuracy of light emission quantity control. It is therefore undesirable to change the current value in a maximum pulse range where the light emission quantity and the heat generation quantity are large. However, the current value of each driving pulse may be low in the minimum pulse range where the light emission quantity is small. In addition, heat generated by the light emitting device itself is also stable. It is therefore possible to control the light quantity with high accuracy when the current value control is used only in a very small light emission range.

The PNM/PDM control, the PWM control and the current value control are changed over in accordance with a target light quantity, and each control is used exclusively from any other control. A controllable dynamic range of light quantity is 13.9:1 in the PNM control ranging from a maximum value 2,000 to a minimum value 144, 5.14:1 in the PDM control ranging from a maximum value 144 to a minimum value 16, 12.2:1 in the PWM control ranging a maximum value 95% to a minimum value 7.8%, and 5:1 in the current value control ranging from a maximum value 1 to a minimum value 0.2. Accordingly, the controllable dynamic range reaches 4358:1 in the combination of these controls.

When an equivalent dynamic range and an equivalent dimming resolution to those described above are obtained only by PNM control, the pulse frequency is about 4 MHz (60Hz×4358×16), and a high-speed drive circuit for the laser light source is required. In the same manner, when the dynamic range and the dimming resolution are obtained only by the PWM control, the pulse width control resolution is about 0.12 ns (1/(120k×4358×16)), and a control circuit operating at 8 GHz is required. Thus, the drive unit for the laser light source can be simplified on a large scale by control with PNM control, PDM control and current value control selected in accordance with each dimming range, as compared with a method for controlling light quantity by PNM control alone or PWM control alone.

Next, another configuration example of an endoscopic apparatus will be described.

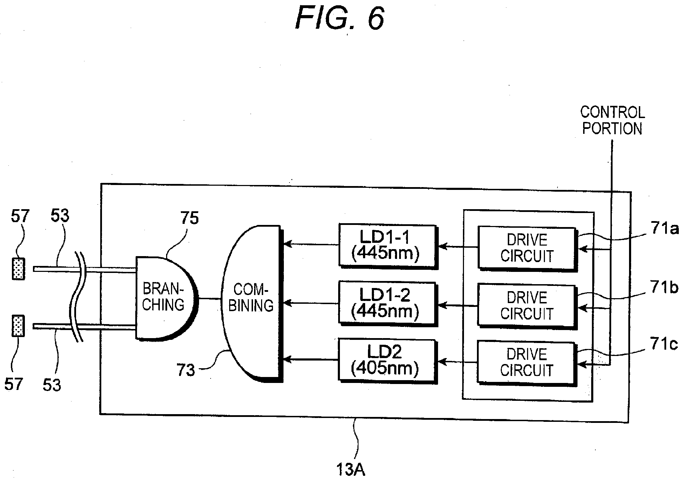

Fig. 6 is a block configuration diagram schematically showing a main portion configuration of a light source unit shown in Fig. 1 and optical paths for guiding light to light irradiation windows.

A light source unit 13A in this configuration example has a laser light source LD1-1 with a center wavelength of 445 nm, a laser light source LD1-2 which is a white light source with the same center wavelength of 445 nm, and a laser light source LD2 which is a narrow-band light source with a center wavelength of 405 nm. In addition, the laser light sources LD1-1, LD1-2 and LD2 are connected to light source drive circuits 71a, 71b and 71c for controlling the laser light sources Lid1-1, LD1-2 and LD2 individually, respectively. Outgoing lights from the laser light sources LD1-1, LD1-2 and LD2 are combined by a combiner 73 and branched into optical fibers 53 and 53 by a coupler 75 so as to outgo to the wavelength conversion portion 57 which is disposed in the endoscope front end portion.

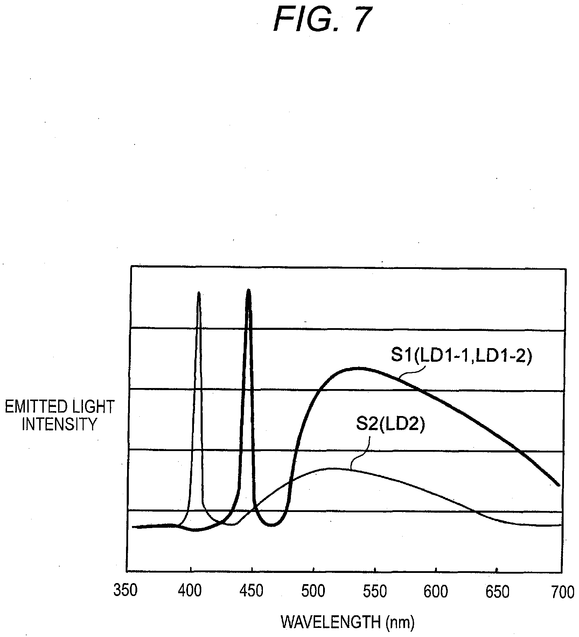

Fig. 7 shows a spectrum of illumination light generated by outgoing light from the light source unit 13A. The laser light sources LD1-1 and LD1-2 emit laser lights of the same spectrum with a center wavelength of 445 nm, so as to excite fluorescent substances of the wavelength conversion portion 57 to generate green to yellow fluorescence. The blue light of the center wavelength 445 nm and the fluorescence from the fluorescent substances are mixed with each other to form white light, which serves as white illumination light.

On the other hand, the laser light source LD2 emits laser light with a center wavelength of 405 nm. Although fluorescence is generated slightly from the wavelength conversion portion 57, most of the emitted light is transmitted through the wavelength conversion light 57. As a result, narrow-band light which is violet is generated to serve as illumination light.

The lights from the laser light sources LD1-1, LD1-2 and LD2 are emitted with equal light quantity from light irradiation windows disposed at a plurality of places in the endoscope front end portion. The following description will be made in the case where pulse lighting is controlled with the light source unit 13A configured thus.

The control portion 61 (see Fig. 1) detects brightness based on RAW data from the imaging device and various photometric modes, and calculates a target light quantity for a next frame, in the same manner as in the aforementioned configuration example shown in Fig. 3. It is preferable that the calculated target light quantity is set in consideration of the following points obtain light emission control values of the individual laser light sources.

### (1) Total Light Quantity Limit

Temperature of each laser light source is detected. When the detected temperature is beyond a specified temperature, correction control is performed to subtract a predetermined value from an intended light quantity control value. On the contrary, when the detected temperature is in a normal temperature range, a predetermined value is added to the light quantity control value which has been controlled to decrease, so that the light quantity control value is brought back to the intended light quantity control value before correction. This correction control is performed to limit heat generated in the endoscope front end portion.

### (2) Individual Difference Correction of Optical Components

In order to correct difference among models of optical components, the light quantity control value of each laser light source after control of the total light quantity of the apparatus is multiplied by a coefficient corresponding to the laser light source. The coefficients of the laser light sources are set to make the total sum of the coefficients constant to thereby keep the total light quantity constant. Since the combiner 73 is used in this configuration, this correction may be dispensed with. When irradiation with light is performed from a plurality of laser light sources individually, the light quantity control values of the laser light sources have to be corrected.

### (3) Light Emission Ratio of Laser Light Source

In order to set the light emission ratio of each laser light source correspondingly to an observation mode of the endoscope, the light quantity control value of the laser light source is multiplied by a coefficient shown in Table 2. The observation mode is changed over from one to another by the control 61, for example, in response to pushing down of an observation mode change button 81 which is one of the operation buttons 27 of the body operation portion 19 shown in Fig. 1.

**[Table 2]**

| | LD1-1 (445 nm) | LD2 (405 nm) | LD1-2 (445 nm) |
|---|---|---|---|
| White Light Emission Mode | 0.50 | 0 | 0.50 |
| Blood Vessel Highlighting Mode | 0.33 | 0.33 | 0.33 |
| Microscopic Blood Vessel Mode | 0.25 | 0.50 | 0.25 |

The white light emission mode in which only the laser light sources LD1-1 and LD1-2 emitting light with the same spectrum are lit to generate white light in cooperation with the wavelength conversion portion 57 is a mode for normal observation in which an affected portion can be observed by irradiation with the white light.

The blood vessel highlighting mode is a mode in which irradiation is performed with white light and narrow-band light with a central wavelength of 405 nm and a bright normal image obtained by irradiation with the white light is combined with a blood vessel image obtained by illumination with the narrow-band light with the central wavelength 405 nm so that a diagnostic image in which the blood vessel image is highlighted selectively is provided. In this mode, an image in which blood vessels can be observed easily even in distant observation can be generated.

The microscopic blood vessel mode is a mode for providing an image in which blood capillaries or microscopic structures in a tissue surface layer are highlighted by illumination with narrow-band light with a central wavelength of 405 nm.

In each of the aforementioned modes, the target light quantity of each light source is corrected. After that, driving pulses are generated by the aforementioned PNM control, PDM control, PWM control and current value control in descending order of target light quantity when each laser light source is driven. Thus, pulse lighting control is performed on each laser light source.

In the white light emission mode, the light quantities intensities of the laser light sources LD1-1 and LD1-2 for generating white illumination light are controlled with the same ratio so that the total light quantity from the two light sources can reach a target light quantity (control of dual-lamp system).

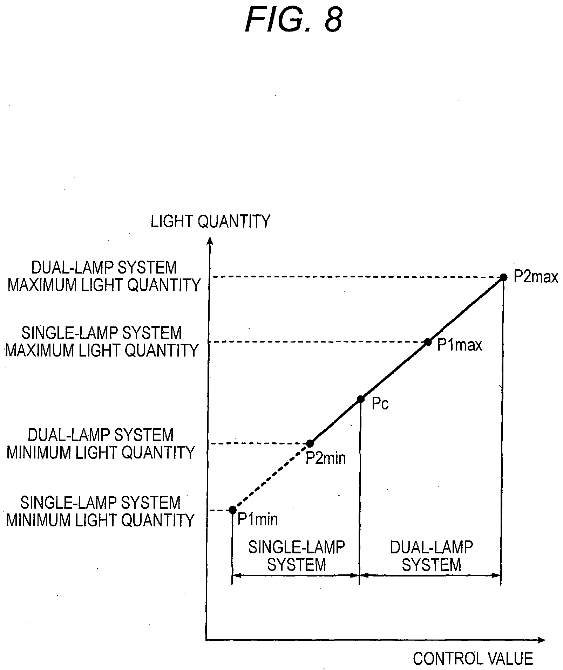

When the target light quantity is low, control of a single-lamp system is performed. That is, only one of the two laser light sources is lit while the other is turned off. Thus, the light quantity control width can be expanded. Fig. 8 shows a light quantity control pattern in combination of a single-lamp system and a dual-lamp system. When the light quantity is controlled by the two light sources lit simultaneously, the light quantity reaches a maximum light quantity with a control value P2max, and reaches a minimum light quantity with a control value P2min. When the light quantity is controlled only by one light source, the light quantity reaches a maximum light quantity with a control value P1 max, and reaches a minimum light quantity with a control value P1min.

Therefore, when the target light quantity is high, the light quantity is controlled in the dual-lamp system to obtain the maximum light quantity. When the target light quantity is low, the light quantity is controlled in the single-lamp system to make the minimum light quantity lower than that in the dual-lamp system. Thus, the light quantity control width can be expanded. In addition, since the lamp system is switched from the dual-lamp system to the single-lamp system when the target light quantity is low, the lighting time of one lamp can be increased to obtain an effect to suppress occurrence of flicker.

In the blood vessel highlighting mode, the light quantities of the three laser light sources LD1-1, LD1-2 and LD2 are controlled with the same ratio so that the total light quantity from the laser light sources can reach a target light quantity.

In the microscopic blood vessel mode, laser light sources with different spectra are lit at a predetermined outgoing light quantity ratio. The outgoing light quantity ratio is set and adjusted by performing control to increase/decrease driving current. The aforementioned PNM control, PDM control, PWM control and current value control are performed while keeping the set outgoing light quantity ratio. In this manner, only the light quantity can be controlled to increase/decrease without disturbing the set outgoing light quantity ratio due to the light quantity control using driving pulses, that is, while keeping the wavelength of outgoing light constant. In addition, there is another advantage that the control of the electronic shutter can be easily operated with each observation mode. Thus, smooth exposure control can be obtained.

According to the endoscopic apparatus configured thus, as described above, it is possible to secure a wider dynamic range with semiconductor light sources than that with a background-art white light source such as a xenon lamp. It is therefore possible to obtain an image with higher image quality. In addition, the light sources can be controlled equivalently to an existing configuration such as a xenon lamp. Thus, an existing processor can be used as it is, so as to form a configuration with an enhanced general purpose. Further, semiconductor light sources have much longer lives than xenon lamps or the like. It is therefore possible to lighten maintenance of the apparatus.

In addition, a laser light source or a light emitting diode with a central wavelength of 360 to 530 nm can be used as the semiconductor light source for illumination with narrow-band light. Thus, it is possible to obtain high luminance illumination light with saved power.

In this manner, the invention is not limited to the aforementioned embodiment, but the invention is intended to cover modifications and applications which can be performed by those skilled in the art based on the description of this specification and well-known techniques. Thus, such modifications and applications are included in the scope of the invention claimed for protection. For example, although description has been made in the case where a laser light source is used as a semiconductor light source by way of example, a configuration using a light emitting diode may be arranged. In addition, light quantity may be controlled by combination of exposure control using an electronic shutter of an imaging unit and light quantity control of a light source.

According to the endoscopic apparatus, a first pulse modulation region where the number of pulses is reduced to shorten the lighting time, a second pulse modulation region where the pulses are thinned to reduce the pulse density, and a third pulse modulation region where the pulse width is reduced, are set in control parameters in descending order of target light quantity. When the target light quantity is high, the control for shortening the lighting time of the light source is made by priority, so as to suppress blurring of a captured image and reduce heat generation. In addition, when the target light quantity is low, there are a plurality of pulses in a predetermined lighting time. Thus, occurrence of flicker can be suppressed. When different kinds of controls are combined, it is possible to secure a wide dynamic range and high dimming resolution.
(2) The endoscopic apparatus according to (1), the light source control unit performs fourth pulse modulation control in which pulse amplitude of each driving pulse whose pulse width is minimized in a region where the third pulse modulation control is performed is reduced when the target light quantity is further lower than a light quantity in the third pulse modulation control.
   According to the endoscopic apparatus, the pulse amplitude is controlled so that the light quantity control width for the minimum light quantity can be expanded.
(3) The endoscopic apparatus according to (1) or (2), further includes a storage unit. The storage unit stores information of control parameters for defining driving pulses of the semiconductor light source for a desired target light quantity. The light source control unit obtains driving pulses in accordance with the inputted target light quantity with reference to the control parameters so as to drive and light the semiconductor light source by using the obtained driving pulses.
   According to the endoscopic apparatus, the control parameters stored in the storage unit are referred to so that the driving pulses can be set rapidly in accordance with the target light quantity. Thus, response can be enhanced.
(4) The endoscopic apparatus according to any one of (1) through (3), the semiconductor light source includes a plurality of light emitting devices which emits light with the same spectrum. The light source control unit generates driving pulses to control and light the light emitting devices individually.
   According to the endoscopic apparatus, the light emitting devices are controlled individually so that the light quantity control width can be expanded.
(5) The endoscopic apparatus according to any one of (1) through (3), the semiconductor light source includes a plurality of light emitting devices which emit light with different spectra from each other. The light source control unit generates driving pulses to control and light the light emitting devices individually.
   According to the endoscopic apparatus, the light emitting devices with different spectra are controlled individually so that light with each spectrum can be emitted with a desired ratio. Also in this case, the dynamic range can be expanded to enhance the light quantity resolution.
(6) The endoscopic apparatus according to (5), the semiconductor light source includes a white light source which generates white light and a narrow-band light source which generates narrow-band light in a predetermined wavelength band. The light source control unit changes an outgoing light quantity ratio between the white light source and the narrow-band light source.
   According to the endoscopic apparatus, the outgoing light quantity ratio between the white light source for normal observation and the narrow-band light source for observation with special light is changed so that an image obtained by the normal observation and an image obtained by the observation with the special light can be combined at a desired ratio to obtain a desired endoscopically diagnostic image.
(7) The endoscopic apparatus according to (6), the narrow-band light source emits narrow-band light with a center wavelength of 360 to 530 nm.
   According to the endoscopic apparatus, the narrow-band light in the visible short wavelength band with a central wavelength of 360 to 530 nm is used so that it is possible to obtain an image in which blood capillaries or microscopic structures in a tissue surface layer are highlighted.
(8) The endoscopic apparatus according to (6) or (7), the white light source includes a laser light source and fluorescent substances which emits light in response to light emitted from the laser light source. The light emitted from the laser light source and the light emitted from the fluorescent substances are mixed to generate the illumination light with a desired spectrum.

According to the endoscopic apparatus, illumination light with a desired spectrum, such as white light, can be obtained stably with high controllability on light quantity by the laser light source which has a long life as a light source.

## Claims

1. An endoscopic apparatus (100) adapted to emit illumination light with a desired light quantity from a front end of an endoscope insertion portion, comprising:
a semiconductor light source (55) adapted to generate the illumination light;
an imaging unit adapted to adjust an exposure time by using an electronic shutter; and
a light source control unit (59) adapted to generate driving pulses to drive and light the semiconductor light source (55) in accordance with an inputted target light quantity;
wherein the light source control unit (59) is adapted to perform first pulse modulation control, second pulse modulation control and third pulse modulation control in descending order of the target light quantity;
in the first pulse modulation control, the number of the driving pulses is reduced to shorten a lighting time of the semiconductor light source (55) to a predetermined lighting time for an exposure time of an electronic shutter within one frame;
in the second pulse modulation control, the driving pulses are thinned at predetermined intervals in a predetermined lighting time in a region where the first pulse modulation control is performed so that pulse density is reduced in the lighting time; and
in the third pulse modulation control, pulse width of each of the driving pulses whose number is minimized in a region where the second pulse modulation control is performed is reduced.

2. The endoscopic apparatus according to Claim 1, wherein:
the light source control unit is adapted to perform fourth pulse modulation control in which pulse amplitude of each driving pulse whose pulse width is minimized in a region where the third pulse modulation control is performed is reduced when the target light quantity is further lower than a light quantity in the third pulse modulation control.

3. The endoscopic apparatus according to Claim 1 or 2, further comprising:
a storage unit that is adapted to store information of control parameters for defining driving pulses of the semiconductor light source for a desired target light quantity; wherein:
the light source control unit is adapted to obtain driving pulses in accordance with the inputted target light quantity with reference to the control parameters so as to drive and light the semiconductor light source by using the obtained driving pulses.

4. The endoscopic apparatus according to any one of Claims 1 through 3, wherein:
the semiconductor light source includes a plurality of light emitting devices are dapted to emit light with the same spectrum; and
the light source control unit is adapted to generate driving pulses to control and light the light emitting devices individually.

5. The endoscopic apparatus according to any one of Claims 1 through 3, wherein:
the semiconductor light source includes a plurality of light emitting devices which are adapted to emit light with different spectra from each other; and
the light source control unit are adapted to generate driving pulses to control and light the light emitting devices individually.

6. The endoscopic apparatus according to Claim 5, wherein:
the semiconductor light source includes a white light source which is adapted to generate white light and a narrow-band light source which generates narrow-band light in a predetermined wavelength band; and
the light source control unit is adapted to change an outgoing light quantity ratio between the white light source and the narrow-band light source.

7. The endoscopic apparatus according to Claim 6, wherein:
the narrow-band light source emits narrow-band light with a center wavelength of 360 to 530 nm.

8. The endoscopic apparatus according to Claim 6 or 7, wherein:
the white light source includes a laser light source and fluorescent substances which are adapted to emit light in response to light emitted from the laser light source; and
the light emitted from the laser light source and the light emitted from the fluorescent substances are mixed to generate the illumination light with a desired spectrum.

## Patentansprüche

1. Endoskopvorrichtung (100), ausgebildet zum Emittieren von Beleuchtungslicht mit einer gewünschten Lichtmenge aus einem vorderen Ende eines Endoskopeinführteils, umfassend:
eine Halbleiterlichtquelle (55), ausgebildet zum Erzeugen des Beleuchtungslichts;
eine Bildaufnahmeeinheit, ausgebildet zum Einstellen einer Belichtungszeit unter Verwendung eines elektronischen Verschlusses; und
eine Lichtquellensteuereinheit (59), ausgebildet zum Erzeugen von Treiberimpulsen zum Treiben und zum Leuchten-Bringen der Halbleiterlichtquelle (55), nach Maßgabe einer eingegebenen Solllichtmenge;
wobei
die Lichtquellensteuereinheit (59) dazu ausgebildet ist, eine erste Pulsmodulationssteuerung, eine zweite Pulsmodulationssteuerung und eine dritte Pulsmodulationssteuerung in absteigender Reihenfolge der Solllichtmenge auszuführen;
bei der ersten Pulsmodulationssteuerung die Anzahl der Treiberimpulse reduziert wird, um die Beleuchtungszeit der Halbleiterlichtquelle (55) auf eine vorbestimmte Beleuchtungszeit für eine Belichtungszeit eines elektronischen Verschlusses innerhalb eines Einzelbilds zu verkürzen;
bei der zweiten Pulsmodulationssteuerung die Treiberimpulse mit vorbestimmten Intervallen in einer vorbestimmten Beleuchtungszeit innerhalb einer Zone ausgedünnt werden, in der die erste Pulsmodulationssteuerung ausgeführt wird, so dass die Pulsdichte in der Beleuchtungszeit reduziert wird; und
bei der dritten Pulsmodulationssteuerung die Pulsbreite jedes der Treiberimpulse, deren Anzahl in einer Zone minimiert ist, in der die zweite Pulsmodulationssteuerung ausgeführt wird, reduziert wird.

2. Endoskopvorrichtung nach Anspruch 1, bei der
die Lichtquellensteuereinheit dazu ausgebildet ist, eine vierte Pulsmodulationssteuerung auszuführen, bei der eine Pulsamplitude jedes Treiberpulses, dessen Pulsbreite in einer Zone minimiert ist, in der die dritte Pulsmodulationssteuerung ausgeführt wird, verringert wird, wenn die Solllichtmenge weiterhin geringer ist als eine Lichtmenge bei der dritten Pulsmodulationssteuerung.

3. Endoskopvorrichtung nach Anspruch 1 oder 2, weiterhin umfassend:
eine Speichereinheit, ausgebildet zum Speichern von Information von Steuerparametern zum Definieren von Treiberimpulsen der Halbleiterlichtquelle für eine gewünschte Solllichtmenge, wobei
die Lichtquellensteuereinheit dazu ausgebildet ist, Treibersignale nach Maßgabe der eingegebenen Solllichtmenge unter Bezugnahme auf die Steuerparameter zu erhalten, um die Halbleiterlichtquelle unter Verwendung der erhaltenen Treiberimpulse zu treiben und zum Licht-Abgeben zu veranlassen.

4. Endoskopvorrichtung nach einem der Ansprüche 1 bis 3, bei der
die Halbleiterlichtquelle mehrere Lichtemissionselemente aufweist, ausgebildet zum Emittieren von Licht mit gleichem Spektrum; und
die Lichtquellensteuereinheit dazu ausgebildet ist, Treiberimpulse zum Steuern und zur Lichtabgabe durch die Lichtemissionsbauelemente individuell zu erzeugen.

5. Endoskopvorrichtung nach einem der Ansprüche 1 bis 3, bei der
die Halbleiterlichtquelle mehrere Lichtemissionsbauelemente enthält, ausgebildet zum Emittieren von Licht unterschiedlicher Spektren; und
wobei die Lichtquellensteuereinheit dazu ausgebildet wird, Treiberimpulse zum individuellen Steuern und Licht-Abgeben durch die Lichtemissionsbauelemente zu erzeugen.

6. Endoskopvorrichtung nach Anspruch 5, bei der
die Halbleiterlichtquelle eine Weißlichtquelle enthält, ausgebildet zum Erzeugen weißen Lichts, und eine schmalbandige Lichtquelle enthält, die schmalbandiges Licht in einem vorbestimmten Wellenlängenband erzeugt; und
die Lichtquellensteuereinheit dazu ausgebildet ist, ein Mengenverhältnis des ausgegebenen Lichts zu ändern zwischen der Weißlichtquelle und der schmalbandigen Lichtquelle.

7. Endoskopvorrichtung nach Anspruch 6, bei der
die schmalbandige Lichtquelle schmalbandiges Licht mit einer Mittenlängenwelle von 360 bis 530 nm emittiert.

8. Endoskopvorrichtung nach Anspruch 6 oder 7, bei der
die Weißlichtquelle eine Laserlichtquelle und fluoreszierende Substanzen enthält, ausgebildet zum Emittieren von Licht ansprechend auf von der Laserlichtquelle emittierten Lichts; und
wobei das von der Laserlichtquelle emittierte Licht und das von den fluoreszierenden Substanzen emittierte Licht gemischt werden, um das Beleuchtungslicht mit einem gewünschten Spektrum zu erzeugen.

## Revendications

1. Appareil endoscopique (100) apte à émettre une lumière d'éclairement présentant une quantité de lumière souhaitée à partir d'une extrémité avant d'une portion d'introduction d'endoscope, comprenant :
une source lumineuse à semi-conducteurs (55) apte à générer la lumière d'éclairement ;
une unité d'imagerie apte à ajuster un temps d'exposition en utilisant un obturateur électronique, et
une unité de commande de source lumineuse (59) apte à générer des impulsions d'attaque destinées à exciter et allumer la source lumineuse à semi-conducteurs (55) en fonction d'une quantité de lumière cible entrée ;
dans lequel
l'unité de commande de source lumineuse (59) est apte à exécuter une première commande de modulation d'impulsions, une deuxième commande de modulation
d'impulsions et une troisième commande de modulation d'impulsions en ordre décroissant de la quantité de lumière cible ;
dans la première commande de modulation d'impulsions, le nombre d'impulsions d'attaque est réduit pour raccourcir un temps d'éclairage de la source lumineuse à semi-conducteurs (55) à un temps d'éclairage prédéterminé pour un temps d'exposition d'un obturateur électronique dans les limites d'une trame ;
dans la deuxième commande de modulation d'impulsions, les impulsions d'attaque sont amincies à des intervalles prédéterminés dans un temps d'éclairage prédéterminé dans une région où la première commande de modulation d'impulsions est réalisée de telle sorte que la densité d'impulsions est réduite dans le temps d'éclairage, et
dans la troisième commande de modulation d'impulsions, on réduit la largeur d'impulsions de chacune des impulsions d'attaque dont le nombre est minimisé dans une région où la deuxième commande de modulation d'impulsions est réalisée.

2. Appareil endoscopique selon la revendication 1, dans lequel
l'unité de commande de source lumineuse est apte à réaliser une quatrième commande de modulation d'impulsions, où on réduit une amplitude d'impulsion de chaque impulsion d'attaque dont la largeur d'impulsion est minimisée dans une région où la troisième commande de modulation d'impulsions est réalisée lorsque la quantité de lumière cible est encore plus basse qu'une quantité de lumière dans la troisième commande de modulation d'impulsions.

3. Appareil endoscopique selon la revendication 1 ou 2, comprenant en outre :
une unité de stockage qui est apte à stocker des informations de paramètres de commande destinés à définir des impulsions d'attaque de la source lumineuse à semi-conducteurs pour une quantité de lumière cible souhaitée, dans lequel
l'unité de commande de source lumineuse est apte à obtenir des impulsions d'attaque en fonction de la quantité de lumière cible entrée avec référence aux paramètres de commande de manière à exciter et allumer la source lumineuse à semi-conducteurs en utilisant les impulsions d'attaque obtenues.

4. Appareil endoscopique selon l'une quelconque des revendications 1 à 3, dans lequel :
la source lumineuse à semi-conducteurs inclut une pluralité de dispositifs luminescents aptes à émettre une lumière présentant le même spectre, et
l'unité de commande de source lumineuse est apte à générer des impulsions d'attaque pour commander et allumer les dispositifs luminescents individuellement.

5. Appareil endoscopique selon l'une quelconque des revendications 1 à 3, dans lequel
la source lumineuse à semi-conducteurs inclut une pluralité de dispositifs luminescents aptes à émettre une lumière présentant des spectres différents les uns des autres, et
l'unité de commande de source lumineuse est apte à générer des impulsions d'attaque pour commander et allumer les dispositifs luminescents individuellement.

6. Appareil endoscopique selon la revendication 5, dans lequel :
la source lumineuse à semi-conducteurs inclut une source de lumière blanche apte à générer une lumière blanche, et une source lumineuse à bande étroite qui génère une lumière à bande étroite dans une bande de longueurs d'onde prédéterminée, et
l'unité de commande de source lumineuse est apte à modifier un rapport de quantité de lumière sortante entre la source de lumière blanche et la source de lumière à bande étroite.

7. Appareil endoscopique selon la revendication 6, dans lequel :
la source de lumière à bande étroite émet une lumière à bande étroite présentant une longueur d'onde centrale allant de 360 à 530 nm.

8. Appareil endoscopique selon la revendication 6 ou 7, dans lequel :
la source de lumière blanche inclut une source de lumière laser et des substances fluorescentes aptes à émettre une lumière en réaction à une lumière émise à partir de la source de lumière laser, et
la lumière émise à partir de la source de lumière laser et la lumière émise à partir des substances fluorescentes sont mélangées pour générer la lumière d'éclairement présentant un spectre souhaité.
